# EUROPEAN PATENT SPECIFICATION

(11) **EP 4 017 383 B1**
(45) Date of publication and mention of the grant of the patent: **17.12.2025**
(21) Application number: 20761784.6
(22) Date of filing: 21.08.2020
(51) Int. Cl.: A61B 17/3207

(54) **ATHERECTOMY DEVICES INCLUDING CUTTING BLADES HAVING DIFFERENT EDGE SHAPES**
ATHEREKTOMIEGERÄTE MIT SCHNEIDKLINGEN MIT UNTERSCHIEDLICHEN KANTENFORMEN
DISPOSITIFS D'ATHÉRECTOMIE COMPRIS DE LAMES DE COUPE AVEC DIFFÉRENTES FORMES DE BORD

(30) Priority: 22.08.2019 US 201962890150 P
(43) Date of publication of application: 29.06.2022
(73) Proprietor: Philips Image Guided Therapy Corporation, San Diego CA 92130 (US); Koninklijke Philips N.V., 5656 AG Eindhoven (NL)
(72) Inventor: ESCUDERO, Paul Q, 5656 AE Eindhoven (NL); ROWE, Douglas, 5656 AE Eindhoven (NL); POMBO, August Christopher, 5656 AE Eindhoven (NL); DE CICCO, Dino, 5656 AE Eindhoven (NL)
(74) Representative: Philips Intellectual Property & Standards
(86) International application number: PCT/EP2020/073555
(87) International publication number: WO 2021/032887

(56) References cited:
- WO-A2-2015/017114
- US-A1- 2007 088 230
- US-A1- 2016 228 146
- US-B2- 10 271 870

## Description

### FIELD OF THE DISCLOSURE

The devices and methods described herein generally relate to treatment of occluded body lumens, such as the removal of occlusive material from a blood vessel or other body parts.

### BACKGROUND

Peripheral and interventional cardiology is a medical specialty that relates to treatment of various forms of cardiovascular disease, including coronary artery disease and peripheral vascular disease. Coronary artery disease and peripheral vascular disease can arise due to the narrowing of the arteries by atherosclerosis (also called arteriosclerosis). Coronary artery disease generally affects arteries of the heart-arteries that carry blood to cardiac muscles and surrounding tissue. Peripheral vascular disease refers to various diseases of the vascular system outside the heart and brain, which carries blood, for example, to the legs.

Atherosclerosis commonly affects the medium and large arteries, and may occur when fat, cholesterol, and other substances build up on the walls of arteries and form fleshy or hard/calcified structures called plaques/lesions. As plaque forms within an arterial wall, the artery may narrow and become less flexible, which may make it more difficult for blood to flow therethrough. In the peripheral arteries, the plaque is typically not localized, but can extend in length along the axis of the artery for as much as 10 mm or more (in some instance up to 400 mm or more).

Pieces of plaque can break off and move through the affected artery to smaller blood vessels, which may in some instances block them and may result in tissue damage or tissue death (embolization). In some cases, the atherosclerotic plaque may be associated with a weakening of the wall of the affected artery, which can lead to an aneurysm. Minimally invasive surgeries may be performed to remove plaque from arteries in an effort to alleviate or help prevent the complications of atherosclerosis.

A number of interventional surgical methodologies may be used to treat atherosclerosis. In balloon angioplasty, for example, a physician may advance a collapsed, intravascular balloon catheter into a narrowed artery, and may inflate the balloon to macerate and/or displace plaque against the vessel wall. A successful angioplasty may help reopen the artery and allow for improved blood flow. Often, balloon angioplasty is performed in conjunction with the placement of a stent or scaffold structure within the artery to help minimize re-narrowing of the artery. Balloon angioplasty, however, can stretch the artery and induce scar tissue formation, while the placement of a stent can cut arterial tissue and also induce scar tissue formation. Scar tissue formation may lead to restenosis of the artery. In some instances, balloon angioplasty can also rip the vessel wall.

Atherectomy is another treatment methodology for atherosclerosis, and involves the use of an intravascular device to mechanically remove (that is, debulk) plaque from the wall of the artery. Atherectomy devices may allow for the removal of plaque from the wall of an artery, reducing the risk of stretching, cutter, or dissecting the arterial wall and causing tissue damage that leads to restenosis. In some instances, atherectomy may be used to treat restenosis by removing scar tissue.

Unfortunately, some atherectomy devices suffer from structural and performance limitations. For example, the cutting elements or assemblies of some atherectomy devices cannot efficiently treat occlusions including heterogeneous mixtures of materials (such as calcium, plaque, collagen, fibrin, and the like). Accordingly, it is desirable to provide improved atherectomy devices and methods.

WO 2015/017114 A2, according to its abstract, describes devices and components for use in performing an atherectomy. Generally, the atherectomy devices may have a handle, a cutter assembly, and a catheter or catheter assembly therebetween. The cutter assembly may include a housing and a cutter comprising a proximal cutter and a distal cutter, each of which may be rotated relative to the atherectomy device to cut occlusive material.

US 2016/228146 A1, according to its abstract, relates to treatment of occluded body lumens. In particular, the present devices and method relate to removal of the occluding material from the blood vessels as well as other body lumens.

US 10271870 B2, according to its abstract, relates to intraluminal procedures, and, more particularly, to a contra-rotating cutting assembly for use with an atherectomy device to remove occlusive material from an occluded lumen, such as a blood vessel or other body lumen. The contra-rotating cutting assembly includes a rotatable housing having a distal end, an opposing proximal end and a lumen extending between the distal and proximal ends. The housing is configured to rotate about a longitudinal axis in a first direction. The cutting assembly further includes a rotatable cutter head positioned within at least a portion of the lumen of the housing and in coaxial alignment with the housing. The cutter head is configured to rotate about the longitudinal axis in a second direction opposite the first direction.

US 2007/088230 A1, according to its abstract, relates to a rotational handle device that rotates a medical instrument for vascular treatment in a tubular form including a tube for insertion by guiding of a medical guide wire inside. The tube includes a helical coil of metal, a tubular tip of metal secured to an end of the helical coil, and a cutting head of a blade shape, formed on the tubular tip, for cutting a lesion upon being rotated. A first rotatable tube rotates with the tube, and has a wire lumen. An externally operable second rotatable tube is secured to the first rotatable tube in an axial direction. An over torque preventing mechanism is disposed between the first and second rotatable tubes, and causes the second rotatable tube to rotate free from the first rotatable tube upon application of torque higher than the predetermined level to the second rotatable tube.

### SUMMARY

The object of the present invention is solved by the subject-matter of the independent claims; further embodiments are incorporated in the dependent claims.

The present disclosure presents an atherectomy device. The atherectomy device includes a handle configured to be manipulated by a user. A catheter is coupled to the handle, and the catheter includes an outer sheath and a drive shaft. The drive shaft is disposed within and rotatable relative to the outer sheath. The atherectomy device further includes a cutter assembly including a housing coupled to and extending distally from the outer sheath. The cutter assembly further includes a proximal cutting element rotatably carried by the housing. The proximal cutting element is coupled to and extends distally from the drive shaft, and the proximal cutting element includes at least one proximal cutting blade. The cutter assembly further includes a distal cutting element that is rotatable with the proximal cutting element relative to the housing. The distal cutting element includes a first distal cutting blade that has a first cutting edge, and the first cutting edge has a first shape. The distal cutting element further includes a second distal cutting blade that has a second cutting edge, and the second cutting edge has a second shape that is different than the first shape.

The atherectomy device according to the previous paragraph, wherein the first cutting edge includes a first proximal portion and a first distal portion, the second cutting edge includes a second proximal portion and a second distal portion, the second proximal portion being disposed radially inwardly relative to the first proximal portion, and the second distal portion being disposed radially outwardly relative to the first distal portion.

The atherectomy device according to any of the previous paragraphs, wherein the first shape is a smooth curve.

The atherectomy device according to any of the previous paragraphs, wherein the first shape and the second shape are smooth curves.

The atherectomy device according to any of the previous paragraphs, wherein the distal cutting element further includes a third distal cutting blade having a third cutting edge.

The atherectomy device according to any of the previous paragraphs, wherein the third cutting edge has a third shape, the third shape being different than the first shape and the second shape.

The atherectomy device according to any of the previous paragraphs, wherein the first cutting edge includes a first proximal portion and a first distal portion, the second cutting edge includes a second proximal portion and a second distal portion, the third cutting edge includes a third proximal portion and a third distal portion, the first proximal portion being disposed radially outwardly relative to the second proximal portion and the third proximal portion, and the second distal portion being disposed radially outwardly relative to the first distal portion and the third distal portion.

The atherectomy device according to any of the previous paragraphs, wherein the first cutting edge further includes a first intermediate portion disposed between the first proximal portion and the first distal portion, the second cutting edge includes a second intermediate portion disposed between the second proximal portion and the second distal portion, the third cutting edge includes a third intermediate portion disposed between the third proximal portion and the third distal portion, the third intermediate portion being disposed radially outwardly relative to the first intermediate portion and the second intermediate portion.

The atherectomy device according to any of the previous paragraphs, wherein the first shape, the second shape, and the third shape are smooth curves.

The atherectomy device according to any of the previous paragraphs, wherein the distal cutting element includes a first number of distal cutting blades, the proximal cutting element includes a second number of proximal cutting blades, and the second number is two times the first number.

The present disclosure also presents an atherectomy device. The atherectomy device includes a handle configured to be manipulated by a user. A catheter is coupled to the handle, and the catheter includes an outer sheath and a drive shaft. The drive shaft is disposed within and rotatable relative to the outer sheath. The atherectomy device further includes a cutter assembly a housing coupled to and extending distally from the outer sheath. The cutter assembly further includes a first cutting blade that is rotatable relative to the housing. The first cutting blade has a first cutting edge, and the first cutting edge has a first shape. The cutter assembly further includes a second cutting blade that is rotatable with the first cutting blade relative to the housing. The second cutting blade has a second cutting edge, and the second cutting edge has a second shape that is different than the first shape. The cutter assembly further includes a third cutting blade that is rotatable with the first cutting blade and the second cutting blade relative to the housing. The third cutting blade has a third cutting edge, and the third cutting edge has a third shape that is different than the first shape and the second shape.

The atherectomy device according to the previous paragraph, wherein the first cutting edge includes a first proximal portion and a first distal portion, the second cutting edge includes a second proximal portion and a second distal portion, the second proximal portion being disposed radially inwardly relative to the first proximal portion, and the second distal portion being disposed radially outwardly relative to the first distal portion.

The atherectomy device according to any of the previous paragraphs, wherein the first cutting edge includes a first proximal portion and a first distal portion, the second cutting edge includes a second proximal portion and a second distal portion, the third cutting edge includes a third proximal portion and a third distal portion, the first proximal portion being disposed radially outwardly relative to the second proximal portion and the third proximal portion, and the second distal portion being disposed radially outwardly relative to the first distal portion and the third distal portion.

The atherectomy device according to any of the previous paragraphs, wherein the first cutting edge further includes a first intermediate portion disposed between the first proximal portion and the first distal portion, the second cutting edge includes a second intermediate portion disposed between the second proximal portion and the second distal portion, the third cutting edge includes a third intermediate portion disposed between the third proximal portion and the third distal portion, the third intermediate portion being disposed radially outwardly relative to the first intermediate portion and the second intermediate portion.

The atherectomy device according to any of the previous paragraphs, wherein the first shape is a smooth curve.

The atherectomy device according to any of the previous paragraphs, wherein the first shape and the second shape are smooth curves.

The atherectomy device according to any of the previous paragraphs, wherein the first shape, the second shape, and the third shape are smooth curves.

The present disclosure also presents an atherectomy device. The atherectomy device includes a handle configured to be manipulated by a user. A catheter is coupled to the handle, and the catheter includes an outer sheath and a drive shaft. The drive shaft is disposed within and rotatable relative to the outer sheath. The atherectomy device further includes a cutter assembly that includes a housing coupled to and extending distally from the outer sheath. The cutter assembly further includes a proximal cutting element rotatably carried by the housing. The proximal cutting element is coupled to and extends distally from the drive shaft, and the proximal cutting element includes at least one proximal cutting blade. The cutter assembly further includes a distal cutting element that is rotatable with the proximal cutting element relative to the housing. The distal cutting element includes a first distal cutting blade that is configured to cut a first profile shape into occlusive material. The distal cutting element further includes a second distal cutting blade that is configured to cut a second profile shape into occlusive material, and the second profile shape is different than the first profile shape.

The atherectomy device according to the previous paragraph, wherein the distal cutting element further includes a third distal cutting blade configured to cut a third profile shape into occlusive material.

The atherectomy device according to any of the previous paragraphs, wherein the third profile shape is different than the first profile shape and the second profile shape.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (for example, X₁ and X₂) as well as a combination of elements selected from two or more classes (for example, Y₁ and Zₒ).

The term "a" or "an" entity refers to one or more of that entity. As such, the terms "a" (or "an"), "one or more" and "at least one" may be used interchangeably herein. It is also to be noted that the terms "comprising", "including", and "having" may be used interchangeably.

The term "means" as used herein shall be given its broadest possible interpretation in accordance with 35 U.S.C. Section 112(f). Accordingly, a claim incorporating the term "means" shall cover all structures, materials, or acts set forth herein, and all of the equivalents thereof. Further, the structures, materials or acts and the equivalents thereof shall include all those described in the summary, brief description of the drawings, detailed description, abstract, and claims themselves.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure may be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
FIG. 1 is a side view of an atherectomy system according to an embodiment of the present disclosure.
FIG. 2A is a detail side view of a distal portion of the atherectomy system of FIG. 1.
FIG. 2B is a detail perspective view of the distal portion of the atherectomy system of FIG. 1.
FIG. 2C is a detail transverse sectional view of the distal portion of the atherectomy system of FIG. 2A.
FIG. 3A is a perspective view of a distal cutting element of the atherectomy system of FIG. 1.
FIG. 3B is a side view of the distal cutting element of FIG. 3A.
FIG. 3C is a cross sectional view of the distal cutting element along line 3C-3C of FIG. 3B.
FIG. 4 is a schematic illustration of cutting profile shapes formed by cutting edges of the distal cutting element of FIG. 3A.
FIG. 5A is a perspective view of a proximal cutting element of the atherectomy system of FIG. 1.
FIG. 5B is a front view of the proximal cutting element of FIG. 5A.

It should be understood that the drawings are not necessarily to scale. In certain instances, details that are not necessary for an understanding of the disclosure or that render other details difficult to perceive may have been omitted. It should be understood, of course, that the disclosure is not necessarily limited to the particular embodiments illustrated herein.

### DETAILED DESCRIPTION

Before any embodiments of the disclosure are explained in detail, it is to be understood that the disclosure is not limited in its application to the details of construction and the arrangement of components set forth in the following description or illustrated in the following drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

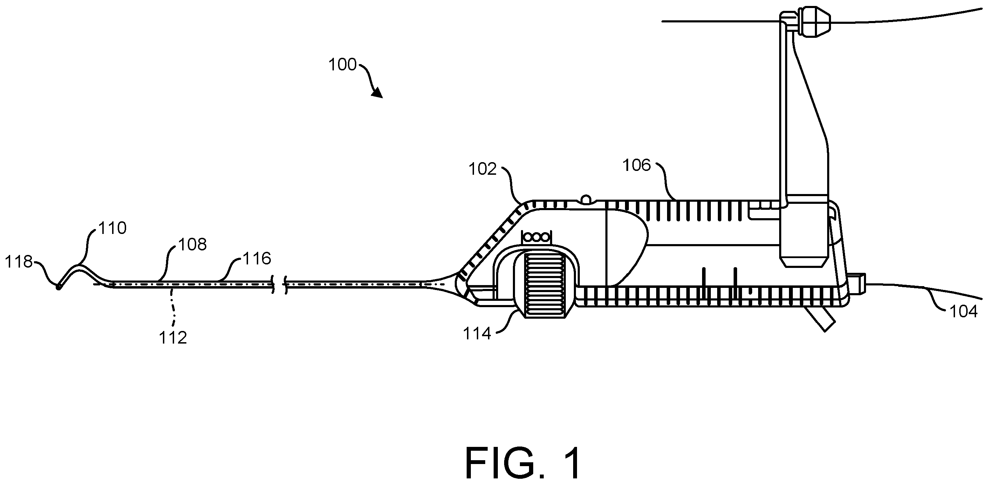

The present disclosure relates generally to devices, systems, and methods for mechanical atherectomy. Referring to FIG. 1, there is shown an exemplary embodiment of the atherectomy systems described herein. The atherectomy system 100 includes an intravascular atherectomy device 102 and a guide wire 104 over which the atherectomy device 102 may be deployed. In some embodiments, the guide wire 104 is silicon-coated or non-coated (bare), or otherwise free of a PTFE coating. Atherectomy systems according to some embodiments of the present disclosure comprise a guide wire 104 that includes a PTFE coating, or atherectomy systems according to some embodiments of the present disclosure lack a guide wire 104.

With continued reference to FIG. 1, the atherectomy device 102 generally includes a handle 106 and a catheter 108. The handle 106 is configured to be grasped and manipulated by a user (for example, a medical professional) during an atherectomy procedure. The catheter 108 is coupled to and extends distally relative to the handle 106. The catheter 108 is configured to be positioned in the vasculature of a subject (for example, a patient) during an atherectomy procedure to facilitate removal of occlusive material (for example, plaque) therefrom. In some embodiments and as illustrated, a distal portion 110 of the catheter 108 has a curved shape or configuration. In some embodiments, the distal portion 110 of the catheter 108 normally has a curved configuration ("normally" being understood as the catheter 108 not being subjected to any external contact forces due to, for example, contact with blood vessel walls) and may be deflected to other configurations. In other embodiments, the distal portion 110 of the catheter 108 normally has a straight shape or configuration and may be deflected to other configurations. In some embodiments, the catheter 108 is selectively rotatable about a catheter rotational axis 112 relative to the handle 106 to facilitate appropriately positioning and or "sweeping" the distal portion 110 of the catheter 108 during an atherectomy procedure. In some embodiments and as illustrated, the handle 106 carries a rotatable knob or dial 114 for selectively rotating the catheter 108 relative to the handle 106. The catheter 108 includes an outer sheath 116, and the outer sheath 116 couples to a cutter assembly 118 that extends distally therefrom. The cutter assembly 118 is described in further detail below.

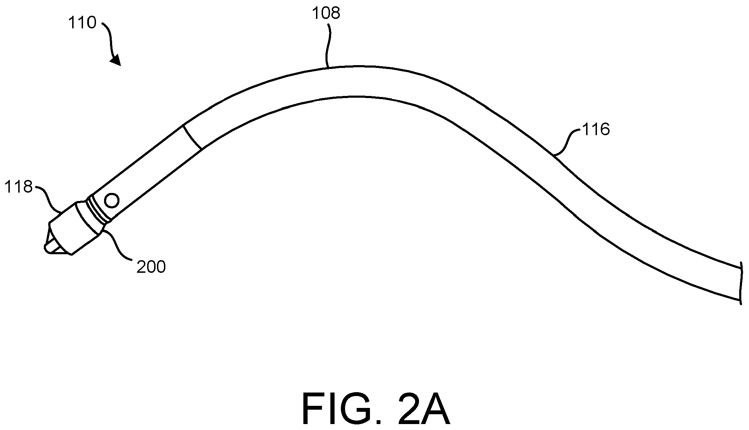

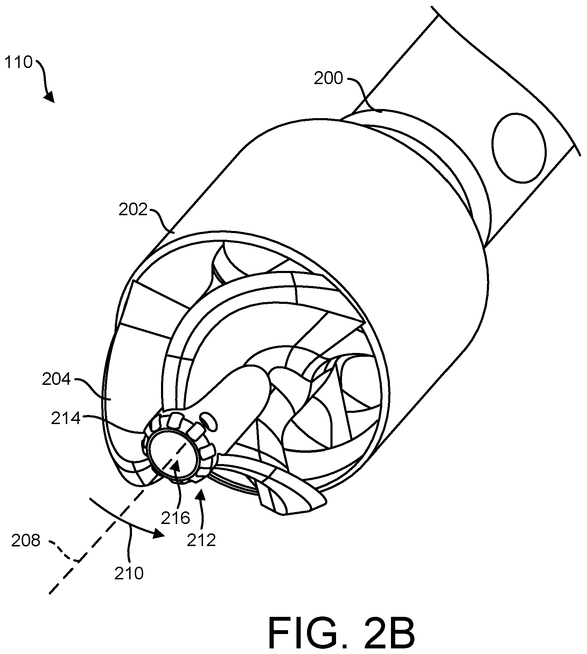

FIGS. 2A-2C illustrate the distal portion 110 of the catheter 108, including, among other components, the outer sheath 116 and the cutter assembly 118. The cutter assembly 118 includes a ferrule 200 that couples to the outer sheath 116 and extends distally therefrom. The cutter assembly 118 further includes a housing 202 that couples to the ferrule 200 and extends distally therefrom. The housing 202 rotatably carries cutting elements. Referring specifically to FIGS. 2B-2C, the housing 202 rotatably carries a first, or distal, cutting element 204 and a second, or proximal, cutting element 206. Rotation of the first cutting element 204 and the second cutting element 206 about a rotation axis 208 in a rotational direction 210 relative to the housing 202 causes the cutting elements 204, 206 to cut occlusive material and convey the occlusive material into the housing 202 (a process also referred to as "debulking").

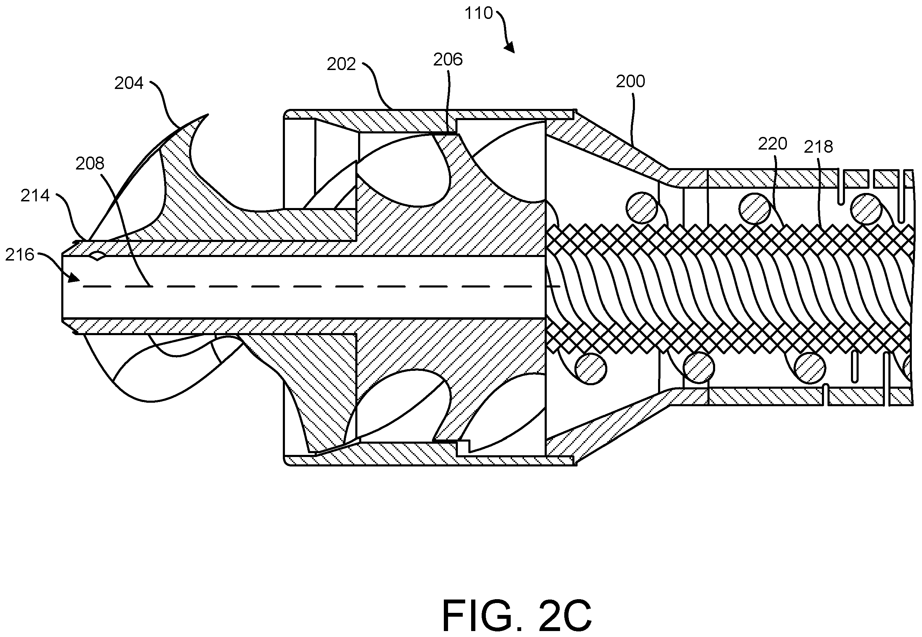

Still referring to FIGS. 2B-2C, the first cutting element 204 generally extends distally from the second cutting element 206 and the housing 202. The first cutting element 204 includes a central opening 212 (see FIG. 2C) for coupling to the second cutting element 206. The second cutting element 206 is generally disposed within the housing 202 and, in some embodiments and as illustrated, may be completely disposed within the housing 202. The second cutting element 206 is also generally disposed proximally from the first cutting element 204, although the second cutting element 206 includes a shaft or stem 214 that is received in the central opening 212. The stem 214 may couple to the first cutting element 204 in various manners. For example, the stem 214 may couple to the first cutting element 204 via welding. In some embodiments and as illustrated, the stem 214 extends distally relative to the first cutting element 204. The stem 214 includes an inner lumen 216 for receiving a guide wire (shown elsewhere).

Referring specifically to FIG. 2C, the atherectomy device 102 further includes a rotatable drive shaft 218 that couples the first cutting element 204 and the second cutting element 206 to a prime mover (for example, a motor carried by the handle 106 - not shown). That is, the prime mover rotates the drive shaft 218, which in turn rotates the first cutting element 204 and the second cutting element 206 to facilitate cutting occlusive material and conveying the occlusive material into the housing 202. In some embodiments, the cutter assembly 118 captures the cut occlusive material from the blood without the use of vacuum aspiration. In other embodiments, vacuum aspiration may assist capture of the cut occlusive material.

With continued reference to FIG. 2C, in some embodiments, the atherectomy device 102 also includes an internal conveyor 220 that is coupled to and rotates with the drive shaft 218. As occlusive material is conveyed into the cutter housing 202 by the first cutting element 204 and the second cutting element 206, the conveyor 220 displaces the cut occlusive material proximally through the catheter 108 for discharge outside the subject's body. In some embodiments, this conveyance may occur without the use of vacuum aspiration assistance. In other embodiments, vacuum aspiration may assist conveyance of the cut occlusive material.

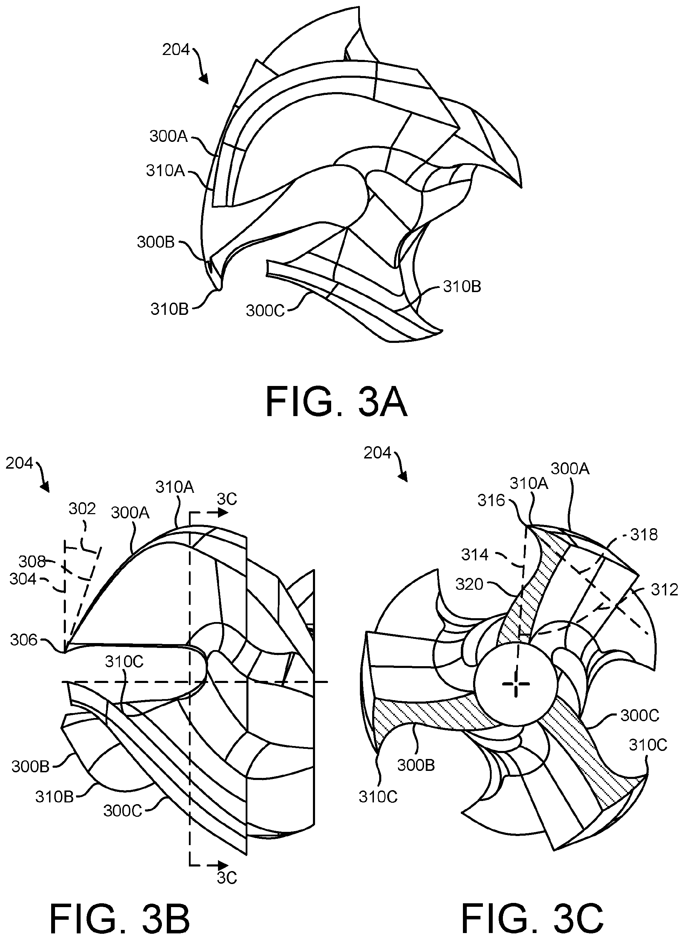

Referring now to FIGS. 3A-3C, the first cutting element 204 includes one or more first, or distal, cutting flutes or blades that extend distally relative to the housing 202. In some embodiments and as illustrated, the first cutting element 204 includes three distal cutting blades 300A, 300B, and 300C. In some embodiments, the first cutting element 204 includes a different number of cutting blades, such as two, four, five, six, seven, eight, nine, ten, or more cutting blades. In some embodiments and as illustrated, one or more of the distal cutting blades 300A, 300B, and 300C extend helically relative to the rotation axis 208 of the first cutting element 204 and the second cutting element 206.

Referring specifically to FIG. 3B, in some embodiments the distal cutting blades 300A, 300B, and 300C have a positive lead-in angle 302. That is, the distal cutting blades 300A, 300B, and 300C have a lead-in angle 302 that is measured between (1) an imaginary line 304 extending perpendicularly relative to the rotation axis 208 at a most distal point 306 of the cutting blades 300A, 300B, and 300C and (2) a tangent 308 to cutting edges 310A, 310B, and 310C of the cutting blades 300A, 300B, and 300C at the most distal point 306 of the cutting blades 300A, 300B, and 300C. In some embodiments, the lead-in angle 302 is in a range of 15 degrees to 55 degrees. In some embodiments, the lead-in angle 302 is in a range of 25 degrees to 45 degrees. In some embodiments, the lead-in angle 302 is in a range of 30 degrees to 40 degrees. In some embodiments, the lead-in angle 302 is substantially 35 degrees (that is, 35 degrees ± 2.5 degrees).

Referring specifically to FIG. 3C, in some embodiments the distal cutting blades 300A, 300B, and 300C have a positive rake angle 312. That is, the distal cutting blades 300A, 300B, and 300C have a rake angle 312 that is measured between an imaginary radius 314 extending from the rotation axis 208 of the first cutting element 204 to a most radially distant edge 316 of the cutting blade 300A, 300B, and 300C and a tangent 318 from an inner face 320 of the cutting blade 300A, 300B, and 300C at the most radially distant edge 316. The rake angle 312 is in the same direction as the rotational direction 210 of the first cutting element 204 and the second cutting element 206 about the rotation axis 208. In some embodiments, the rake angle 312 is in a range of 30 degrees to 80 degrees. In some embodiments, the rake angle 312 is in a range of 35 degrees to 75 degrees. In some embodiments, the rake angle 312 is in a range of 40 degrees to 70 degrees. In some embodiments, the rake angle 312 is in a range of 45 degrees to 65 degrees. In some embodiments, the rake angle 312 is in a range of 50 degrees to 60 degrees. In some embodiments, the rake angle 312 is substantially 55 degrees (that is, 55 degrees ± 2.5 degrees).

In some embodiments, one or more of the distal cutting blades 300A, 300B, and 300C have cutting edges 310A, 310B, and 310C with different shapes. Such different cutting edges 310A, 310B, and 310C are configured to cut different portions of occlusive material, which facilitates cutting relatively small portions of occlusive material compared to cutting elements with cutting edges 310A, 310B, and 310C having the same shape. In some cases, cutting relatively small portions of occlusive material is relatively efficient and facilitates treating hard occlusions, such as occlusions including calcium. Furthermore, such different cutting edges 310A, 310B, and 310C facilitate efficiently treating occlusions including heterogeneous mixtures of materials (such as calcium, plaque, collagen, fibrin, and the like).

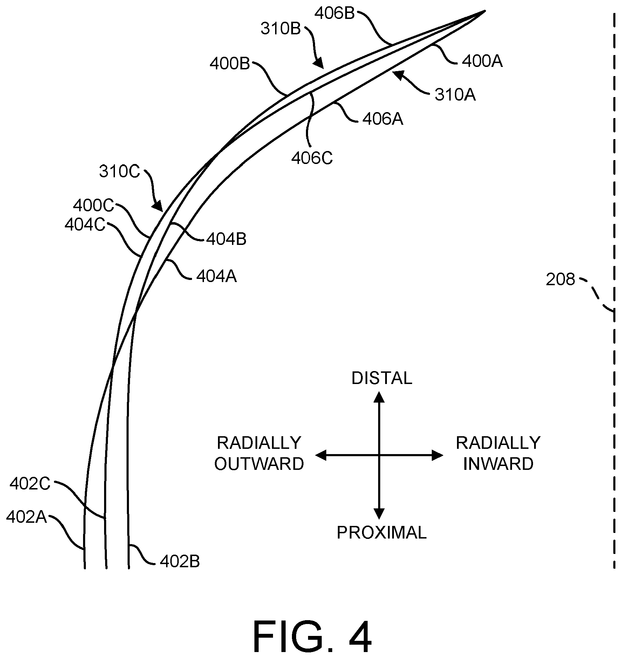

Referring specifically to FIG. 4, the cutting profile shape 400A, 400B, and 400C formed by the different cutting edges 310A, 310B, and 310C of the cutting blades 300A, 300B, and 300C, respectively, are illustrated. Stated another way, FIG. 4 illustrates the paths along which each cutting edge 310A, 310B, and 310C intersects an imaginary transverse plane as the cutting element 204 rotates about the rotation axis 208. Stated yet another way, Fig. 4 schematically illustrates edges of occlusive material cut by the cutting edges 310A, 310B, and 310C from a transverse sectional view.

The following paragraphs describe specific shapes of the cutting edges 310A, 310B, and 310C and the cutting profile shape 400A, 400B, and 400C. Only the cutting edges 310A, 310B, and 310C are referenced for brevity, but it is understood that the description applies to both the cutting edges 310A, 310B, and 310C and the cutting profile shape 400A, 400B, and 400C. In some embodiments and as illustrated, each of the cutting edges 310A, 310B, and 310C includes a portion that is disposed further radially outwardly than portions of the other cutting edges 310A, 310B, and 310C at the same position along the rotation axis 208. More specifically, the first cutting edge 310A includes a first proximal portion 402A, a first intermediate portion 404A, and a first distal portion 406A, the second cutting edge 310B includes a second proximal portion 402B, a second intermediate portion 404B, and a second distal portion 406B, and the third cutting edge 310C includes a third proximal portion 402C, a third intermediate portion 404C, and a third distal portion 406C. The first proximal portion 402A is disposed radially outwardly relative to the second proximal portion 402B and the third proximal portion 402C, the second distal portion 406B is disposed radially outwardly relative to the first distal portion 406A and the third distal portion 406C, and the third intermediate portion 404C is disposed radially outwardly relative to the first intermediate portion 404A and the second intermediate portion 404B.

In some embodiments and as illustrated, the cutting edges 310A, 310B, and 310C have shapes that are smooth curves. That is, the cutting profile shapes 400A, 400B, and 400C lack sharp points.

In some embodiments, the cutting edges 310A, 310B, and 310C may have different shapes than those described above. For example, in some embodiments one or more of the cutting edges 310A, 310B, and 310C includes multiple portions that are disposed further radially outwardly than portions of the other cutting edges 310A, 310B, and 310C at the same axial positions. As another example, in some embodiments one or more of the cutting edges 310A, 310B, and 310C have shapes that are not smooth curves. That is, one or more of the cutting profile shapes 400A, 400B, and 400C includes one or more sharp points. As yet another example, in some embodiments one or more of the cutting edges 310A, 310B, and 310C includes saw-tooth type features and/or a hemispherical pattern, which may facilitate pulverizing dense calcium.

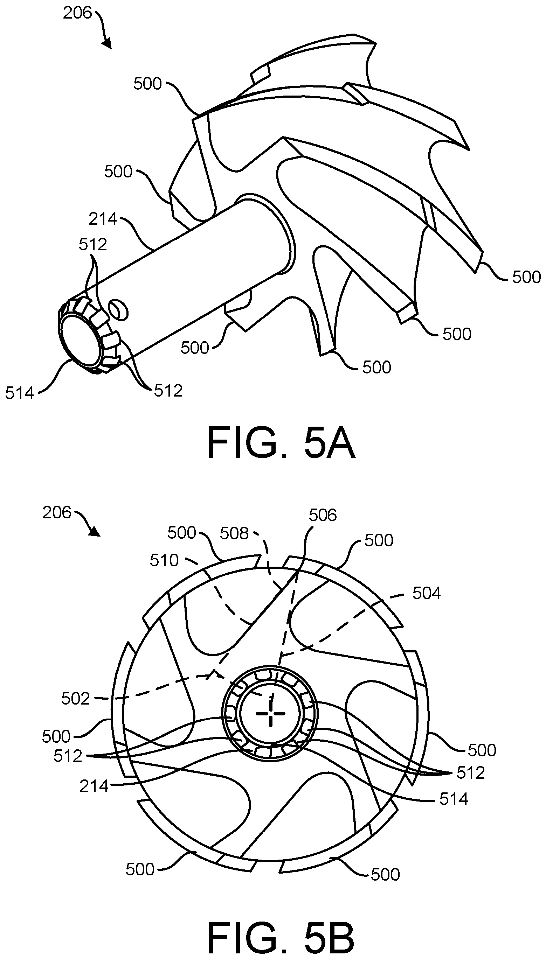

Referring now to FIGS. 5A-5B, the second cutting element 206 includes one or more second, or proximal, cutting flutes or blades 500. In some embodiments, the second cutting element 206 has two times the number of blades 500 as the first cutting element 204. In some embodiments and as illustrated, the second cutting element 206 includes six cutting blades 500. In some embodiments and as illustrated, the proximal cutting blades 500 extend helically relative to the rotation axis 208 of the first cutting element 204 and the second cutting element 206. In some embodiments and referring specifically to FIG. 5B, the proximal cutting blades 500 have a negative rake angle 502. Stated another way, the proximal cutting blades 500 have a rake angle 502 that is measured between an imaginary radius 504 extending from the rotation axis 208 of the second cutting element to a most radially distant edge 506 of the cutting blade 500 to a tangent 508 from an inner face 510 of the cutting blade 500 at the most radially distant edge 506. The rake angle 502 is in the opposite direction as the rotational direction 210 of the first cutting element 204 and the second cutting element 206 about the rotation axis 208. Stated yet another way, the inner face 510 the proximal cutting blades 500 may slant outward or forward of the cutting edge. In some embodiments, the rake angle 502 is in a range of 5 degrees to 45 degrees (also referred to as -5 degrees to -45 degrees). In some embodiments, the rake angle 502 is in a range of 6 degrees to 35 degrees (also referred to as -6 degrees to -35 degrees). In some embodiments, the rake angle 502 is in a range of 8 degrees to 24 degrees (also referred to as - 8 degrees to -24 degrees). In some embodiments, the rake angle 502 is substantially 16 degrees (that is, 16 degrees ± 2.5 degrees; also referred to as substantially -16 degrees (that is, -16 degrees ± 2.5 degrees)).

In some embodiments, the positive rake angle 312 of the first cutting element 204 and the negative rake angle 502 of the second cutting element 206 facilitate improved cutting efficiency and/or inhibit clogging of occlusive material in the housing 202. More specifically, in some embodiments the positive rake angle 312 of the first cutting element 204 facilitates cutting and conveying occlusive material toward the second cutting element 206 and the negative rake angle 502 of the second cutting element 206 facilitates displacing occlusive materially radially outwardly toward the housing 202 and proximally, thereby inhibiting clogging of occlusive material in the housing 202.

In some embodiments and as illustrated, the cutting stem 214 also includes one or more cutting features 512 that facilitate fragmenting occlusive material into small particles to be captured and removed by the atherectomy system 100. In some embodiments and as illustrated, the cutting stem 214 includes nine cutting features 512. In other embodiments, the cutting stem 214 includes a different number of cutting features 512 (for example, one, two, three, four, five, six, seven, eight, ten, or more cutting features 512). In some embodiments, the cutting features 512 are negative features (for example, channels formed on the surface of the cutting stem 214, as illustrated, or depressions formed on the surface of the cutting stem 214). In some embodiments, the cutting features 512 are positive features (for example, ridges or protrusions extending from the surface of the cutting stem 214). In some embodiments and as illustrated, the cutting features 512 extend substantially proximally from a leading end 514 of the cutting stem 214. In some embodiments, the cutting features 512 are disposed apart from the leading end 514 and/or do not extend proximally along the stem 214.

The foregoing discussion has been presented for purposes of illustration and description. The foregoing is not intended to limit the disclosure to the form or forms disclosed herein. In the foregoing Summary for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the disclosure, for example, as may be within the skill and knowledge of those in the art, after understanding the present disclosure. The scope of the invention is defined by the appended claims.

## Claims

1. An atherectomy device (102), comprising:
a handle (106) configured to be manipulated by a user;
a catheter (108) coupled to the handle, the catheter comprising an outer sheath (116) and a drive shaft (218), wherein the drive shaft is disposed within and rotatable relative to the outer sheath; and
a cutter assembly (118) comprising:
a housing (202) coupled to and extending distally from the outer sheath;
a proximal cutting element (206) rotatably carried by the housing, the proximal cutting element being coupled to and extending distally from the drive shaft, the proximal cutting element comprising at least one proximal cutting blade (500);
a distal cutting element (204) being rotatable with the proximal cutting element relative to the housing, the distal cutting element comprising:
a first distal cutting blade (300) having a first cutting edge (310A), the first cutting edge having a first shape; and
a second distal cutting blade (300) having a second cutting edge (310B), the second cutting edge having a second shape, the second shape being different than the first shape;
**characterised in that**
the first cutting edge comprises a first proximal portion (402A) and a first distal portion (406A), the second cutting edge comprises a second proximal portion (402B) and a second distal portion (406B), the second proximal portion being disposed radially inwardly relative to the first proximal portion, and the second distal portion being disposed radially outwardly relative to the first distal portion.

2. The atherectomy device of claim 1, wherein the first shape is a smooth curve.

3. The atherectomy device of claim 1, wherein the first shape and the second shape are smooth curves.

4. The atherectomy device of claim 1, wherein the distal cutting element further comprises a third distal cutting blade having a third cutting edge (310C).

5. The atherectomy device of claim 4, wherein the third cutting edge has a third shape, the third shape being different than the first shape and the second shape.

6. The atherectomy device of claim 5, wherein the third cutting edge (310C) comprises a third proximal portion and a third distal portion, the first proximal portion being disposed radially outwardly relative to the second proximal portion and the third proximal portion, and the second distal portion being disposed radially outwardly relative to the first distal portion and the third distal portion.

7. The atherectomy device of claim 6, wherein the first cutting edge further comprises a first intermediate portion (404A) disposed between the first proximal portion and the first distal portion, the second cutting edge comprises a second intermediate portion (404B) disposed between the second proximal portion and the second distal portion, the third cutting edge comprises a third intermediate portion (404C) disposed between the third proximal portion and the third distal portion, the third intermediate portion being disposed radially outwardly relative to the first intermediate portion and the second intermediate portion.

8. The atherectomy device of claim 7, wherein the first shape, the second shape, and the third shape are smooth curves.

9. The atherectomy device of claim 1, wherein the distal cutting element includes a first number of distal cutting blades, the proximal cutting element includes a second number of proximal cutting blades, and the second number is two times the first number.

10. An atherectomy device (102), comprising:
a handle (106) configured to be manipulated by a user;
a catheter (108) coupled to the handle, the catheter comprising an outer sheath and a drive shaft, wherein the drive shaft is disposed within and rotatable relative to the outer sheath; and
a cutter assembly (118) comprising:
a housing (202) coupled to and extending distally from the outer sheath;
a first cutting blade (300A) being rotatable relative to the housing, the first cutting blade having a first cutting edge, the first cutting edge having a first shape;
a second cutting blade (300B) being rotatable with the first cutting blade relative to the housing, the second cutting blade having a second cutting edge, the second cutting edge having a second shape, the second shape being different than the first shape; and
a third cutting blade (300C) being rotatable with the first cutting blade and the second cutting blade relative to the housing, the third cutting blade having a third cutting edge, the third cutting edge having a third shape, the third shape being different than the first shape and the second shape;
**characterised in that**
the first cutting edge comprises a first proximal portion and a first distal portion, the second cutting edge comprises a second proximal portion and a second distal portion, the second proximal portion being disposed radially inwardly relative to the first proximal portion, and the second distal portion being disposed radially outwardly relative to the first distal portion.

11. The atherectomy device of claim 10, wherein the first cutting edge comprises a first proximal portion and a first distal portion, the second cutting edge comprises a second proximal portion and a second distal portion, the third cutting edge comprises a third proximal portion and a third distal portion, the first proximal portion being disposed radially outwardly relative to the second proximal portion and the third proximal portion, and the second distal portion being disposed radially outwardly relative to the first distal portion and the third distal portion.

12. The atherectomy device of claim 11, wherein the first cutting edge further comprises a first intermediate portion disposed between the first proximal portion and the first distal portion, the second cutting edge comprises a second intermediate portion disposed between the second proximal portion and the second distal portion, the third cutting edge comprises a third intermediate portion disposed between the third proximal portion and the third distal portion, the third intermediate portion being disposed radially outwardly relative to the first intermediate portion and the second intermediate portion.

13. The atherectomy device of claim 10, wherein the first shape is a smooth curve.

14. The atherectomy device of claim 10, wherein the first shape and the second shape are smooth curves.

15. The atherectomy device of claim 10, wherein the first shape, the second shape, and the third shape are smooth curves.

## Patentansprüche

1. Atherektomievorrichtung (102), umfassend:
einen Griff (106), der dazu konfiguriert ist, von einem Benutzer gehandhabt zu werden;
einen Katheter (108), der mit einem Griff gekoppelt ist, wobei der Katheter eine Außenhülle (116) und eine Antriebswelle (218) umfasst, wobei die Antriebswelle in der Außenhülle angeordnet und relativ zu dieser drehbar ist; und
eine Schneidanordnung (118), umfassend:
ein Gehäuse (202), das mit der Außenhülle gekoppelt ist und sich distal von dieser erstreckt;
ein proximales Schneidelement (206), das drehbar vom Gehäuse getragen wird, wobei das proximale Schneidelement mit der Antriebswelle gekoppelt ist und sich distal von dieser erstreckt, wobei das proximale Schneidelement mindestens eine proximale Schneidklinge (500) umfasst;
ein distales Schneidelement (204), das mit dem proximalen Schneidelement relativ zum Gehäuse drehbar ist, wobei das distale Schneidelement Folgendes umfasst:
eine erste distale Schneidklinge (300), die eine erste Schneidkante (310A) aufweist, wobei die erste Schneidkante eine erste Form aufweist; und
eine zweite distale Schneidklinge (300), die eine zweite Schneidkante (310B) aufweist, wobei die zweite Schneidkante eine zweite Form aufweist und sich die zweite Form von der ersten Form unterscheidet;
**dadurch gekennzeichnet, dass**
die erste Schneidkante einen ersten proximalen Abschnitt (402A) und einen ersten distalen Abschnitt (406A) umfasst, die zweite Schneidkante einen zweiten proximalen Abschnitt (402B) und einen zweiten distalen Abschnitt (406B) umfasst, der zweite proximale Abschnitt relativ zum ersten proximalen Abschnitt radial nach innen angeordnet ist und der zweite distale Abschnitt relativ zum ersten distalen Abschnitt radial nach außen angeordnet ist.

2. Atherektomievorrichtung nach Anspruch 1, wobei die erste Form eine glatte Kurve ist.

3. Atherektomievorrichtung nach Anspruch 1, wobei die erste Form und die zweite Form glatte Kurven sind.

4. Atherektomievorrichtung nach Anspruch 1, wobei das distale Schneidelement weiter eine dritte distale Schneidklinge umfasst, die eine dritte Schneidkante (310C) aufweist.

5. Atherektomievorrichtung nach Anspruch 4, wobei die dritte Schneidkante eine dritte Form aufweist, wobei sich die dritte Form von der ersten und der zweiten Form unterscheidet.

6. Atherektomievorrichtung nach Anspruch 5, wobei die dritte Schneidkante (310C) einen dritten proximalen Abschnitt und einen dritten distalen Abschnitt umfasst, der erste proximale Abschnitt relativ zum zweiten proximalen Abschnitt und zum dritten proximalen Abschnitt radial nach außen angeordnet ist und der zweite distale Abschnitt relativ zum ersten distalen Abschnitt und zum dritten distalen Abschnitt radial nach außen angeordnet ist.

7. Atherektomievorrichtung nach Anspruch 6, wobei die erste Schneidkante weiter einen ersten Zwischenabschnitt (404A) umfasst, der zwischen dem ersten proximalen Abschnitt und dem ersten distalen Abschnitt angeordnet ist, die zweite Schneidkante einen zweiten Zwischenabschnitt (404B) umfasst, der zwischen dem zweiten proximalen Abschnitt und dem zweiten distalen Abschnitt angeordnet ist, die dritte Schneidkante einen dritten Zwischenabschnitt (404C) umfasst, der zwischen dem dritten proximalen Abschnitt und dem dritten distalen Abschnitt angeordnet ist, wobei der dritte Zwischenabschnitt relativ zum ersten Zwischenabschnitt und zum zweiten Zwischenabschnitt radial nach außen angeordnet ist.

8. Atherektomievorrichtung nach Anspruch 7, wobei die erste Form, die zweite Form und die dritte Form glatte Kurven sind.

9. Atherektomievorrichtung nach Anspruch 1, wobei das distale Schneidelement eine erste Anzahl distaler Schneidklingen beinhaltet, das proximale Schneidelement eine zweite Anzahl proximaler Schneidklingen beinhaltet und die zweite Anzahl doppelt so groß wie die erste Anzahl ist.

10. Atherektomievorrichtung (102), umfassend:
einen Griff (106), der dazu konfiguriert ist, von einem Benutzer gehandhabt zu werden;
einen Katheter (108), der mit einem Griff gekoppelt ist, wobei der Katheter eine Außenhülle und eine Antriebswelle umfasst, wobei die Antriebswelle in der Außenhülle angeordnet und relativ zu dieser drehbar ist; und
eine Schneidanordnung (118), umfassend:
ein Gehäuse (202), das mit der Außenhülle gekoppelt ist und sich distal von dieser erstreckt;
eine erste Schneidklinge (300A), die relativ zum Gehäuse drehbar ist, wobei die erste Schneidklinge eine erste Schneidkante aufweist, wobei die erste Schneidkante eine erste Form aufweist;
eine zweite Schneidklinge (300B), die mit der ersten Schneidklinge relativ zum Gehäuse drehbar ist, wobei die zweite Schneidklinge eine zweite Schneidkante aufweist, wobei die zweite Schneidkante eine zweite Form aufweist, wobei sich die zweite Form von der ersten Form unterscheidet; und
eine dritte Schneidklinge (300C), die mit der ersten Schneidklinge und der zweiten Schneidklinge relativ zum Gehäuse drehbar ist, wobei die dritte Schneidklinge eine dritte Schneidkante aufweist, wobei die dritte Schneidkante eine dritte Form aufweist, wobei sich die dritte Form von der ersten Form und der zweiten Form unterscheidet;
**dadurch gekennzeichnet, dass**
die erste Schneidkante einen ersten proximalen Abschnitt und einen ersten distalen Abschnitt umfasst, die zweite Schneidkante einen zweiten proximalen Abschnitt und einen zweiten distalen Abschnitt umfasst, der zweite proximale Abschnitt relativ zum ersten proximalen Abschnitt radial nach innen angeordnet ist und der zweite distale Abschnitt relativ zum ersten distalen Abschnitt radial nach außen angeordnet ist.

11. Atherektomievorrichtung nach Anspruch 10, wobei die erste Schneidkante einen ersten proximalen Abschnitt und einen ersten distalen Abschnitt umfasst, die zweite Schneidkante einen zweiten proximalen Abschnitt und einen zweiten distalen Abschnitt umfasst, die dritte Schneidkante einen dritten proximalen Abschnitt und einen dritten distalen Abschnitt umfasst, der erste proximale Abschnitt relativ zum zweiten proximalen Abschnitt und zum dritten proximalen Abschnitt radial nach außen angeordnet ist und der zweite distale Abschnitt relativ zum ersten distalen Abschnitt und zum dritten distalen Abschnitt radial nach außen angeordnet ist.

12. Atherektomievorrichtung nach Anspruch 11, wobei die erste Schneidkante weiter einen ersten Zwischenabschnitt umfasst, der zwischen dem ersten proximalen Abschnitt und dem ersten distalen Abschnitt angeordnet ist, die zweite Schneidkante einen zweiten Zwischenabschnitt umfasst, der zwischen dem zweiten proximalen Abschnitt und dem zweiten distalen Abschnitt angeordnet ist, die dritte Schneidkante einen dritten Zwischenabschnitt umfasst, der zwischen dem dritten proximalen Abschnitt und dem dritten distalen Abschnitt angeordnet ist, wobei der dritte Zwischenabschnitt relativ zum ersten Zwischenabschnitt und zum zweiten Zwischenabschnitt radial nach außen angeordnet ist.

13. Atherektomievorrichtung nach Anspruch 10, wobei die erste Form eine glatte Kurve ist.

14. Atherektomievorrichtung nach Anspruch 10, wobei die erste Form und die zweite Form glatte Kurven sind.

15. Atherektomievorrichtung nach Anspruch 10, wobei die erste Form, die zweite Form und die dritte Form glatte Kurven sind.

## Revendications

1. Dispositif d'athérectomie (102), comprenant :
une poignée (106) configurée pour être manipulée par un utilisateur ;
un cathéter (108) couplé à la poignée, le cathéter comprenant une gaine extérieure (116) et un arbre d'entraînement (218), dans lequel l'arbre d'entraînement est disposé à l'intérieur de et peut tourner par rapport à la gaine extérieure ; et
un ensemble de coupe (118) comprenant :
un boîtier (202) couplé à et s'étendant de manière distale depuis la gaine extérieure ;
un élément de coupe proximal (206) porté de manière rotative par le boîtier, l'élément de coupe proximal étant couplé à et s'étendant de manière distale depuis l'arbre d'entraînement, l'élément de coupe proximal comprenant au moins une lame de coupe proximale (500) ;
un élément de coupe distal (204) pouvant tourner avec l'élément de coupe proximal par rapport au boîtier, l'élément de coupe distal comprenant :
une première lame de coupe distale (300) présentant une première arête de coupe (310A), la première arête de coupe présentant une première forme ; et
une deuxième lame de coupe distale (300) présentant une deuxième arête de coupe (310B), la deuxième arête de coupe présentant une deuxième forme, la deuxième forme étant différente de la première forme ;
**caractérisé en ce que**
la première arête de coupe comprend une première partie proximale (402A) et une première partie distale (406A), la deuxième arête de coupe comprend une deuxième partie proximale (402B) et une deuxième partie distale (406B), la deuxième partie proximale étant disposée radialement vers l'intérieur par rapport à la première partie proximale, et la deuxième partie distale étant disposée radialement vers l'extérieur par rapport à la première partie distale.

2. Dispositif d'athérectomie selon la revendication 1, dans lequel la première forme est une courbe lisse.

3. Dispositif d'athérectomie selon la revendication 1, dans lequel la première forme et la deuxième forme sont des courbes lisses.

4. Dispositif d'athérectomie selon la revendication 1, dans lequel l'élément de coupe distal comprend en outre une troisième lame de coupe distale présentant une troisième arête de coupe (310C).

5. Dispositif d'athérectomie selon la revendication 4, dans lequel la troisième arête de coupe présente une troisième forme, la troisième forme étant différente de la première forme et de la deuxième forme.

6. Dispositif d'athérectomie selon la revendication 5, dans lequel la troisième arête de coupe (310C) comprend une troisième partie proximale et une troisième partie distale, la première partie proximale étant disposée radialement vers l'extérieur par rapport à la deuxième partie proximale et à la troisième partie proximale, et la deuxième partie distale étant disposée radialement vers l'extérieur par rapport à la première partie distale et à la troisième partie distale.

7. Dispositif d'athérectomie selon la revendication 6, dans lequel la première arête de coupe comprend en outre une première partie intermédiaire (404A) disposée entre la première partie proximale et la première partie distale, la deuxième arête de coupe comprend une deuxième partie intermédiaire (404B) disposée entre la deuxième partie proximale et la deuxième partie distale, la troisième arête de coupe comprend une troisième partie intermédiaire (404C) disposée entre la troisième partie proximale et la troisième partie distale, la troisième partie intermédiaire étant disposée radialement vers l'extérieur par rapport à la première partie intermédiaire et à la deuxième partie intermédiaire.

8. Dispositif d'athérectomie selon la revendication 7, dans lequel la première forme, la deuxième forme et la troisième forme sont des courbes lisses.

9. Dispositif d'athérectomie selon la revendication 1, dans lequel l'élément de coupe distal inclut un premier nombre de lames de coupe distales, l'élément de coupe proximal inclut un second nombre de lames de coupe proximales, et le second nombre est deux fois supérieur au premier nombre.

10. Dispositif d'athérectomie (102), comprenant :
une poignée (106) configurée pour être manipulée par un utilisateur ;
un cathéter (108) couplé à la poignée, le cathéter comprenant une gaine extérieure et un arbre d'entraînement, dans lequel l'arbre d'entraînement est disposé à l'intérieur de et peut tourner par rapport à la gaine extérieure ; et
un ensemble de coupe (118) comprenant :
un boîtier (202) couplé à et s'étendant de manière distale à partir de la gaine extérieure ;
une première lame de coupe (300A) pouvant tourner par rapport au boîtier, la première lame de coupe présentant une première arête de coupe, la première arête de coupe présentant une première forme ;
une deuxième lame de coupe (300B) pouvant tourner avec la première lame de coupe par rapport au boîtier, la deuxième lame de coupe présentant une deuxième arête de coupe, la deuxième arête de coupe présentant une deuxième forme, la deuxième forme étant différente de la première forme ; et
une troisième lame de coupe (300C) pouvant tourner avec la première lame de coupe et la deuxième lame de coupe par rapport au boîtier, la troisième lame de coupe présentant une troisième arête de coupe, la troisième arête de coupe présentant une troisième forme, la troisième forme étant différente de la première forme et de la deuxième forme ;
**caractérisé en ce que**
la première arête de coupe comprend une première partie proximale et une première partie distale, la deuxième arête de coupe comprend une deuxième partie proximale et une deuxième partie distale, la deuxième partie proximale étant disposée radialement vers l'intérieur par rapport à la première partie proximale, et la deuxième partie distale étant disposée radialement vers l'extérieur par rapport à la première partie distale.

11. Dispositif d'athérectomie selon la revendication 10, dans lequel la première arête de coupe comprend une première partie proximale et une première partie distale, la deuxième arête de coupe comprend une deuxième partie proximale et une deuxième partie distale, la troisième arête de coupe comprend une troisième partie proximale et une troisième partie distale, la première partie proximale étant disposée radialement vers l'extérieur par rapport à la deuxième partie proximale et à la troisième partie proximale, et la deuxième partie distale étant disposée radialement vers l'extérieur par rapport à la première partie distale et à la troisième partie distale.

12. Dispositif d'athérectomie selon la revendication 11, dans lequel la première arête de coupe comprend en outre une première partie intermédiaire disposée entre la première partie proximale et la première partie distale, la deuxième arête de coupe comprend une deuxième partie intermédiaire disposée entre la deuxième partie proximale et la deuxième partie distale, la troisième arête de coupe comprend une troisième partie intermédiaire disposée entre la troisième partie proximale et la troisième partie distale, la troisième partie intermédiaire étant disposée radialement vers l'extérieur par rapport à la première partie intermédiaire et à la deuxième partie intermédiaire.

13. Dispositif d'athérectomie selon la revendication 10, dans lequel la première forme est une courbe lisse.

14. Dispositif d'athérectomie selon la revendication 10, dans lequel la première forme et la deuxième forme sont des courbes lisses.

15. Dispositif d'athérectomie selon la revendication 10, dans lequel la première forme, la deuxième forme et la troisième forme sont des courbes lisses.
